(19) Europäisches Patentamt
European Patent Office
Office européen des brevets

(11) Veröffentlichungsnummer: **0 129 499**
**B1**

(12) # EUROPÄISCHE PATENTSCHRIFT

(45) Veröffentlichungstag der Patentschrift: **09.12.87**

(51) Int. Cl.⁴: **C 07 J 41/00**, A 61 K 31/565 // C07J71/00

(21) Anmeldenummer: **84730062.1**

(22) Anmeldetag: **13.06.84**

(54) **13alpha-Alkylgonane, deren Herstellung und diese enthaltende pharmazeutische Präparate.**

(30) Priorität: **15.06.83 DE 3321826**
**04.04.84 DE 3413036**

(43) Veröffentlichungstag der Anmeldung: **27.12.84 Patentblatt 84/52**

(45) Bekanntmachung des Hinweises auf die Patenterteilung: **09.12.87 Patentblatt 87/50**

(84) Benannte Vertragsstaaten: **AT BE CH DE FR GB IT LI LU NL SE**

(56) Entgegenhaltungen:
**EP - A - 0 057 115**
**FR - A - 2 377 418**

**CHEMICAL ABSTRACTS, Band 90, Nr. 13, 26. März 1979, Seiten 619-630, Nr. 104208y, Columbus, Ohio, US**
**BERICHTE DER DEUTSCHEN CHEMISCHEN GESELLCHAFT, Jahrgang 74, Nr. 7, 9. Juli 1941, Seiten 1308-1312, Berlin, DE; E. KOENIGS u.a.: "Zur Kenntnis des Einwirkunsproduktes von Hydrazin auf 4-Chlor-chinaldin"**

(73) Patentinhaber: **SCHERING AKTIENGESELLSCHAFT**
***Berlin und Bergkamen,***
**Müllerstrasse 170/178 Postfach 65 03 11,**
**D-1000 Berlin 65 (DE)**

(72) Erfinder: **Neef, Günter, Dr., Darmstädter Strasse 9, D-1000 Berlin 15 (DE)**
Erfinder: **Sauer, Gerhard, Dr., Königsbacher Zeile 47 A, D-1000 Berlin 28 (DE)**
Erfinder: **Wiechert, Rudolf, Prof. Dr., Petzower Strasse 8 A, D-1000 Berlin 39 (DE)**
Erfinder: **Beier, Sybille, Dr., Uhlandstrasse 121, D-1000 Berlin 31 (DE)**
Erfinder: **Elger, Walter, Dr., Schorlemer Allee 12 B, D-1000 Berlin 33 (DE)**
Erfinder: **Henderson, David, Dr., Jahnstrasse 17, D-1000 Berlin 28 (DE)**
Erfinder: **Rohde, Ralph, Dr., Schwatlowstrasse 4, D-1000 Berlin 45 (DE)**

EP 0 129 499 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 13α-Alkylgonane der allgemeinen Formel I [insert pages 1a und 1b] ein Verfahren zu deren Herstellung, diese Verbindungen enthaltende pharmazeutische Präparate sowie die neuen 13-Episteroide der allgemeinen Formel III insert p. 1c als Zwischenprodukte.

Die Verbindungen der allgemeinen Formel I besitzen eine starke Affinität zum Gestagenrezeptor, ohne selbst gestagene Aktivität zu besitzen. Sie sind kompetitive Antagonisten des Progesterons (Anti-Gestagene) und sind zur Auslösung von Aborten geeignet, da sie das zur Aufrechterhaltung der Schwangerschaft erforderliche Progesteron vom Rezeptor verdrängen. Die Verbindungen sind deshalb wertvoll und interessant im Hinblick auf ihre Verwendung zur postcoitalen (p.c.) Fertilitätskontrolle.

Entsprechende Verbindungen in der Estranreihe werden bereits als antigestagen wirksame Verbindungen in Fertility and Sterility 40 (1982), Seite 253, beschrieben.

Die bisher bekannten Struktur-Wirkungsbeziehungen für kompetitive Progesteron-Antagonisten deuteten darauf hin, dass eine 1,3-diaxiale Anordnung von 11β-Arylrest und 13β-Alkylgruppe für die Entfaltung antigestagener Aktivität zwingend erforderlich sei. Um so überraschender ist die starke und selektive antagonistische Wirkung der 13α-Alkylgonane des Typs I, der eine völlig andere molekulare Topographie im Vergleich zur Estran-Reihe aufweist.

Zur Kennzeichnung der antigestagenen Wirkung der erfindungsgemässen Verbindungen wurde die abortive Wirkung in einer frühen Phase post nidationem (Experiment I) und in einer fortgeschrittenen Phase post nidationem (Experiment II) untersucht.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d 1 p.c.).

Die Behandlung der Tiere mit der jeweils zu testenden Substanz bzw. dem Lösungsmittel erfolgte nach der Nidation der Blastocysten von d 5 p.c. bis d 7 p.c. (Experiment I) bzw. d 13 p.c. bis d 15 p.c. (Experiment II). An d 9 p.c. bzw. d 17 p.c. wurden die Tiere getötet und die Uteri auf Implantate und Resorptionsstellen hin untersucht. Von allen Uteri wurden Fotos angefertigt. Das Fehlen von Implantaten wurde als Abort gewertet.

Als Antigestagene wurden untersucht:

A: 11β-(4-Dimethylaminophenyl)-17β-hydroxy- 17α-propinyl-4,9-estradien-3-on (Referenzsubstanz).

B: 11β-(4-Dimethylaminophenyl)-17β-hydroxy-17α- (3-hydroxypropyl)-13α-methyl-4,9-gonadien-3-on (gemäss Erfindung).

C: 11β-(4-Dimethylaminophenyl)-17α-hydroxy-17β- (3-hydroxypropyl)-13α-methyl-4,9-gonadien-3-on (gemäss Erfindung).

Die Testsubstanzen wurden in einem Benzylbenzoat-Rizinusöl-Gemisch (Verhältnis 1:4) gelöst. Das Vehikelvolumen pro Einzeldosis betrug 0,2 ml. Die Behandlung erfolgte subcutan (s.c.).

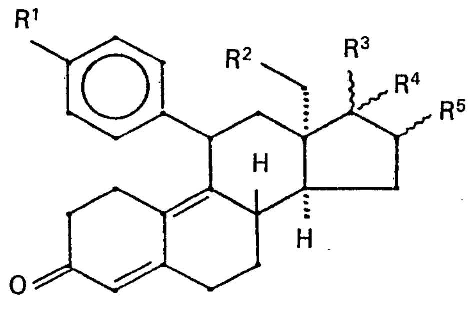

(I),

worin

$R^1$ $-N<^{R^I}_{R^{II}}$ mit $R^I$ und $R^{II}$ in der Bedeutung von Wasserstoff oder Alkyl mit 1 bis 4 Kohlenstoffatomen oder $R^I$ und $R^{II}$ unter Einschluss von N in der Bedeutung eines gesättigten 5- oder 6-gliedrigen Ringes, wobei im Ring ausser N noch ein weiteres Heteroatom wie O, N, S enthalten sein kann, sowie die entsprechenden tertiären N-Oxide und die Säureadditionssalze,

$-OR^{III}$ mit $R^{III}$ in der Bedeutung von Methyl, Ethyl, Propyl, Methoxyphenyl, Allyl oder β-Dimethylaminoethyl,

$R^2$ ein Wasserstoffatom, eine Methyl- oder Ethylgruppe,

$R^3$ $-(CH_2)_n-CH_3$ mit n=0–4,

$-(CH_2)_n-CH_2-O(S)R^{IV}$ mit n=0–4 und $R^{IV}$ in der Bedeutung von Wasserstoff oder Alkyl mit 1 bis 4 Kohlenstoffatomen,

$-CH=CH-(CH_2)_n-OR^V$ mit n=1–4 und $R^V$ in der Bedeutung von Wasserstoff, Alkyl oder Alkanoyl mit jeweils 1 bis 4 Kohlenstoffatomen,

$-C\equiv C-X$ mit X in der Bedeutung von Wasserstoff, Alkyl mit 1 bis 4 Kohlenstoffatomen oder Halogen,

$-(CH_2)_n-CH_2CN$ mit n=0–3 oder

$-\overset{\overset{O}{\|}}{C}-CH_2Y$ mit Y in der Bedeutung von Wasserstoff oder $OR^V$ mit $R^V$ in der oben angegebenen Bedeutung,

$R^4$ Hydroxy, Alkyloxy oder Alkanoyloxy mit jeweils 1 bis 4 Kohlenstoffatomen oder

$R^3/R^4$ 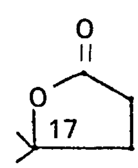

wobei $R^3$ in α-Stellung und $R^4$ in β-Stellung oder $R^3$ in β-Stellung und $R^4$ in α-Stellung zum Steroidgerüst stehen, und

$R^5$ ein Wasserstoffatom oder eine α- oder β-ständige Alkylgruppe mit 1 bis 4 Kohlenstoffatomen bedeuten. Weitere Gegenstände sind

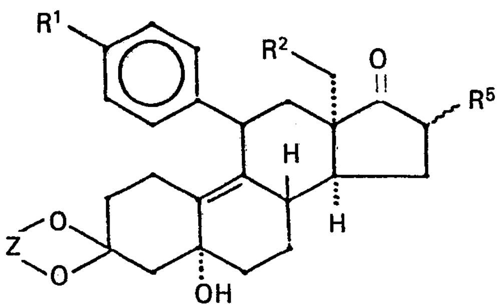

(III),

worin

$R^1$ $-N{<}^{R^{I}}_{R^{II}}$ mit $R^I$ und $R^{II}$ in der Bedeutung von Wasserstoff oder Alkyl mit 1 bis 4 Kohlenstoffatomen oder $R^I$ und $R^{II}$ unter Einschluss von N in der Bedeutung eines gesättigten 5- oder 6-gliedrigen Ringes, wobei im Ring ausser N noch ein weiteres Heteroatom wie O, N, S enthalten sein kann, sowie die entsprechenden tertiären N-Oxide und die Säureadditionssalze,

$-OR^{III}$ mit $R^{III}$ in der Bedeutung von Methyl, Ethyl, Propyl, Methoxyphenyl, Allyl oder β-Dimethylaminoethyl,

$R^2$ ein Wasserstoffatom, eine Methyl- oder Ethylgruppe,

$R^5$ ein Wasserstoffatom oder eine α- oder β-ständige Alkylgruppe mit 1 bis 4 Kohlenstoffatomen und

Z eine Ethylen- oder 2,2-Dimethylpropylengruppe bedeuten.

*Tabelle 1 (Experiment I)*

Abortive Wirkung in einer frühen Phase der Schwangerschaft von Ratten

Behandlung von d 5 p.c. bis d 7 p.c., Autopsie d 9 p.c.

| Verbindung | Dosis mg/Tier/Tag s.c. | Abortrate n Abort/ n Gesamt | (%) |
|---|---|---|---|
| | 30,0 | 4/4 | (100) |
| | 10,0 | – | – |
| A | 3,0 | 4/4 | (100) |
| | 1,0 | 2/4 | ( 50) |
| | 0,3 | 0/4 | ( 0) |
| | 0,1 | 0/4 | ( 0) |
| | 10,0 | 4/4 | (100) |
| | 3,0 | 4/4 | (100) |
| B | 1,0 | 4/4 | (100) |
| | 0,3 | 0/4 | ( 0) |
| | 0,1 | 0/4 | ( 0) |
| | 10,0 | 4/4 | (100) |
| | 3,0 | 4/4 | (100) |
| C | 1,0 | 4/4 | (100) |
| | 0,3 | 0/4 | ( 0) |
| | 0,1 | 0/4 | ( 0) |
| Lösungsmittel als Kontrolle: 0,2 ml Benzylbenzoat + Rizinusöl (1:4) | – | 0/4 | ( 0) |

n = 4 Ratten/Gruppe

*Tabelle 2 (Experiment II)*

Abortive Wirkung in einer fortgeschrittenen Phase der Schwangerschaft von Ratten

Behandlung mit 3,0 mg/d. s.c. Antigestagen (AG) von d 13 p.c. bis d 15 p.c., Autopsie d 17 p.c.

% Kompletter Aborte = leere Nidationsstellen

| | | | | |
|---|---|---|---|---|
| 100 | | | | |
| 90 | | AG | | |
| 80 | | ( ) Zahl der Ratten | | |
| 70 | | | | |
| 60 | | | | 52,3% |
| 50 | | | | (6) |
| 40 | | | 35,4% | |
| 30 | | | (6) | |
| 20 | 0% | 3,5% | | |
| 10 | (10) | (6) | | |
| 0 | | | | |
| | Kontrolle | A | B | C |

Die erfindungsgemässen Verbindungen B und C wirken bei der frühgraviden Ratte in Dosierungen ≧ 1,0 mg/d voll abortiv (Abortrate: 4/4). Im Gegensatz dazu zeigt die Referenzsubstanz A maximale abortauslösende (antigestagene) Wirkung erst bei Dosen von ≧ 3,0 mg/d (Tabelle 1).

In einer forgeschrittenen Phase der Schwangerschaft (d 13–15 p.c.) beträgt der Prozentsatz kompletter Aborte bei 3tätiger s.c.-Gabe von 3,0 mg/d für B 35,4%, für C 52,3% und für die Vergleichssubstanz A 3,5% (Tabelle 2).

Die 13α-Alkylgonane der allgemeinen Formel I können in Form pharmazeutischer Präparate Verwendung finden. Die Herstellung der Präparate erfolgt nach an sich bekannten Methoden der Galenik durch Mischen mit organischem oder anorganischem inertem Trägermaterial, welches für die enterale, perkutane oder parenterale Applikation geeignet ist.

Die Dosierung der erfindungsgemässen Wirkstoffe liegt beim Menschen bei etwa 10 bis 1000 mg pro Tag.

Alkylgruppen in $R^I$ und $R^{II}$ der allgemeinen Formel I sollen 1 bis 4 Kohlenstoffatome enthalten, wobei die Methyl- und Ethylgruppe bevorzugt sind. Die Gruppe $N{<}^{R^{I}}_{R^{II}}$ steht auch für einen gesättigten 5- oder 6-gliedrigen Ring, der ausser C-Atome und N noch ein weiteres Heteroatom wie O, N oder S enthalten kann, beispielsweise genannt seien der Pyrrolidino-, Piperidino-, Piperazino-, Morpholino-, Oxa- und Thiazolidino sowie Thiadiazolidinoring.

Unter $N<^{R^I}_{R^{II}}$ sollen auch die entsprechenden tertiären N-Oxide verstanden werden, wie zum Beispiel Dimethylamino-N-Oxid, Pyrrolidino-, Piperidino-, Piperazino- usw. N-Oxid.

Die in $R^{IV}$ und $R^V$ enthaltenen Alkyl- und Alkanoylgruppen sollen jeweils 1 bis 4 Kohlenstoffatome haben, wobei Methyl, Ethyl und Acetyl, Propionyl bevorzugt sind.

Die neuen 13α-Alkylgonane der allgemeinen Formel I werden erfindungsgemäss nach dem Verfahren gemäss Anspruch 13 hergestellt.

Durch Bestrahlung mit ultraviolettem Licht werden die 13β-Alkylsteroide der allgemeinen Formel II in guter Ausbeute in die 13-Episteroide (13α-Alkylsteroide) der allgemeinen Formel III umgewandelt.

Die gute Ausbeute an Umwandlungsprodukt ist überraschend. Zwar ist lange bekannt, dass 17-Oxosteroide der Normalreihe durch UV-Bestrahlung in die 13-Episteroide überführbar sind (A. Butenandt et al., Ber. Deutsch. Chem. Ges. 74, 1308 (1941)); man erhielt jedoch stets Gemische aus Ausgangsmaterial und epimerisierter Verbindung, die Bestrahlungszeiten betrugen mehrere Stunden, und die Ausbeuten waren ausserordentlich niedrig. Daher ist auch die Suche nach einem alternativen, chemischen Zugang in die 13-Epi-Reihe immer noch aktuell, wie eine jüngere Arbeit von Barton et al., J.C.S. Perkin I, 2163 (1977) zeigt. Diese Alternative ist jedoch zur Herstellung von Verbindungen des Typs I nicht geeignet. Es wurde gefunden, dass die Bestrahlung von Verbindungen des Typs II unter bestimmten Bedingungen einen wesentlich günstigeren Verlauf nimmt als in der Reihe 11-unsubstituierter 17-Oxosteroide. So betragen die durchschnittlichen Bestrahlungszeiten nur noch 10–30 Minuten, und die Ausbeuten an 13-Episteroid liegen zwischen 60–80%. Die Bestrahlungsprodukte können gegebenenfalls ohne chromatographische Reinigung weiter umgesetzt werden. Wesentliche Voraussetzung für ein gutes Ergebnis ist die richtige Wahl des Lösungsmittels, die Konzentration des zu bestrahlenden Substrats und das extakte Einhalten der Bestrahlungsdauer. Diese Parameter sind für jedes neue Substrat gesondert zu ermitteln.

Die Bestrahlung wird mit dem vollen Licht einer Hg-Hochdrucklampe in der Quartzglas-Apparatur durchgeführt. Die Temperatur der Reaktionslösungen wird auf etwa 25 °C eingestellt, die Konzentration der Lösungen beträgt 0,1–1,0 Gewichtsprozent. Als Lösungsmittel werden bevorzugt Tetrahydrofuran und Dioxan verwendet, jedoch können auch unpolare aprotische Solventien wie Hexan, Cyclohexan, Benzol, Toluol und deren Gemische angewendet werden. Die Bestrahlungsdauer beträgt etwa10–15 Minuten.

Die auf diese Weise erhaltenen 13-Episteroide III werden nach den üblichen Verfahren durch nucleophile Addition an das 17-Keton und Folgereaktionen in die Endverbindung der allgemeinen Formel I überführt. Die nucleophile Addition an III verläuft im allgemeinen unter Bildung beider möglicher, isomerer Formen an C-17, die jedoch durch Chromatographie oder fraktionierte Kristallisation leicht trennbar sind. In vielen Fällen sind beide Isomere pharmakologisch wirksam, wenn auch Unterschiede in der Wirkungsstärke bestehen können.

Die nucleophile Addition von Acetylen (Ethin) oder Propin erfolgt mit Hilfe eines den Rest $-C \equiv CH$ oder $-C \equiv C-CH_3$ abgebenden Mittels. Solche Mittel sind zum Beispiel Alkalimetallacetylide, wie zum Beispiel Kalium- und Lithiumacetylid bzw. -methylacetylid.

Die metallorganische Verbindung kann auch in situ gebildet und mit dem 17-Keton der Formel III zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die 17-Ethinyl-17-hydroxy-Verbindungen lassen sich in alkoholischer Lösung unter Quecksilbersalzkatalyse hydratisieren zu den 17-Acetyl-17-hydroxy-Verbindungen (Chem. Ber. 111 (1978) 3086–3093).

Die Einführung von 3-Hydroxypropan bzw. 3-Hydroxypropen in 17-Stellung erfolgt durch Umsetzung des 17-Ketons mit metallierten Derivaten des Propargylalkohols, zum Beispiel mit 1-Lithium-3-tetrahydropyran-2'-yloxy-propin-1, zu den 17-(3-Hydroxy-1-propinyl)-17-hydroxy-Verbindungen, die anschliessend zu den 17-(3-Hydroxypropyl- bzw. 3-Hydroxypropenyl-)-17-hydroxy-Verbindungen hydriert werden. Die Hydrierung muss unter Bedingungen durchgeführt werden, die ausschliesslich den Angriff an der C-C-Dreifachbindung gewährleisten, ohne die tetrasubstituierte 9(10)-Doppelbindung abzusättigen. Das gelingt zum Beispiel bei der Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung der homologen Hydroxyalkan- und Hydroxyalken-Gruppen der allgemeinen Formel $-CH=CH-(CH_2)_nOH$ oder $-CH_2-CH_2-(CH_2)_nOH$ erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134, und H.O. House: Modern Synthetic Reactions 1972, Seite 19). Als

desaktivierte Edelmetallkatalysatoren kommen beispielsweise 10% Palladium auf Bariumsulfat in Gegenwart eines Amins oder 5% Palladium auf Calciumcarbonat unter Zusatz von Blei(II)-acetat in Frage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941), 216), mit Natriumamid in flüssigem Ammoniak (J. Chem. Soc. 1955, 3558), mit Lithium in niedermolekularen Aminen (J. Am. Chem. Soc. 77 (1955) 3378), mit Boranen (J. Am. Chem. Soc. 93 (1971) 3395 und 94 (1971) 6560) mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) (4358) sowie allgemein die Reduktion durch Einwirkung von Übergangsmetallverbindungen unter Wechsel der Oxydationsstufe.

Werden Endprodukte der Formel I gewünscht mit $R^3/R^4$ in der Bedeutung 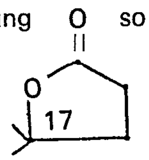 so werden die 17-(3-Hydroxypropyl)-17-hydroxy-Verbindungen in an sich bekannter Weise oxydiert. Die Bedingungen bei der Oxydation sind abhängig von der Natur des Substituenten $R^1$ in Formel I. Ist $R^1$ zum Beispiel eine Dialkylaminogruppe, so sind Chromsäurereagenzien zur Oxydation ungeeignet, da diese primär an der Dialkylaminogruppe angreifen. In diesen Fällen müssen Oxydationsmittel, wie Silbercarbonat/Celite (Fetizon-Reagenz; M. Fetizon und M. Golfier, Compt. rend. 267 (1968) 900) oder Platin/Sauerstoff (H. Muxfeldt et al., Angewandte Chemie, Int. Ed. 1 (1962) 157) Verwendung finden. Ist $R^1$ dagegen eine Alkoxygruppe, so können auch Oxydationsmittel, wie Jones'-Reagenz, Chromsäure-Pyridin, Pyridiniumdichromat oder Pyridiniumchlorochromat verwendet werden.

Der Aufbau der 17-Cyanmethylseitenkette erfolgt in an sich bekannter Weise aus dem 17-Keton der allgemeinen Formel III, zum Beispiel über das 17-Spiroepoxid und Spaltung des Spiroepoxids mit HCN gemäss Z. Chem. 18 (1978) 259–260.

Die Einführung der Cyanalkanseitenkette $n(CH_2)_n-CH_2CN$ mit n=1–3 erfolgt zum Beispiel durch Addition von $Li-(CH_2)_n-CH_2CN$ an das 17-Keton.

Auch die Einführung der 17-Hydroxyacetylseitenkette erfolgt nach an sich bekannten Methoden, beispielsweise nach der in J. Org. Chem. 47 (1982), 2993–2995, beschriebenen Methode.

Freie Hydroxygruppen in 17-Stellung können in an sich bekannter Weise verestert oder verethert werden.

Nach Umsetzung (nucleophiler Addition) des 17-Ketons werden die Verbindungen der Formel III zur Wasserabspaltung unter Ausbildung der 4(5)-Doppelbindung und zur gleichzeitigen Ketalspaltung und Entfernung gegebenenfalls vorhandener weiterer mit Säure abspaltbarer Schutzgruppen mit Säure oder einem sauren Ionenaustauscher behandelt.

Die saure Behandlung erfolgt in an sich bekannter Weise, indem man die Verbindung der Formel III, die eine 3-Ketalgruppe und eine 5α-Hydroxygruppe und anstelle der C 17-Carbonylfunktion eine gegebenenfalls O-geschützte 17-(3-Hydroxypropyl)-Gruppe enthält, in einem mit Wasser mischbaren Lösungsmittel, wie wässrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure oder eine organische Säure, wie Essigsäure, so lange einwirken lässt, bis Wasser abgespalten ist und Schutzgruppen entfernt sind. Die Umsetzung, die bei Temperaturen von 0 bis 100 °C abläuft, kann auch mit einem sauren Ionenaustauscher vorgenommen werden. Der Verlauf der Umsetzung kann mit analystischen Methoden, beispielsweise durch Dünnschichtchromatographie entnommener Proben, verfolgt werden.

Zusätzlich zu den in den Beispielen 1 bis 7 beschriebenen Verbindungen sind folgende Verbindungen bevorzugt:

11β-(4-Dimethylaminophenyl)-17α-(3-hydroxypropyl)- 17β-hydroxy-13α-methyl-4,9-gonadien-3-on-N-oxid.

17α-Ethynyl-17β-hydroxy-13α-methyl-11β-(4-piperidinophenyl)-4,9-gonadien-3-on.

17β-Ethynyl-17α-hydroxy-13α-methyl-11β-(4-piperidinophenyl)-4,9-gonadien-3-on.

11β-[4-(2-Dimethylaminoethoxy)- phenyl]-17α-hydroxy-17β- (3-hydroxypropyl)-13α-methyl-4,9-gonadien-3-on.

11β-(4-Ethoxyphenyl)-17α-hydroxy-17β-(3-hydroxypropyl)-13α-methyl-4,9-gonadien-3-on.

11β-(4-Dimethylaminophenyl)-17α-hydroxy-13α-methyl-17β- n-propyl-4,9-gonadien-3-on.

11β-(4-Dimethylaminophenyl)-17α-hydroxy-17β-(3-methoxypropyl)- 13α-methyl-4,9-gonadien-3-on.

11β-(4-Dimethylaminophenyl)-17β- (ethoxymethyl)-17α-hydroxy-13α-methyl-4,9-gonadien-3-on.

17β-(3-Acetoxy-1Z-propenyl)-11β- (4-dimethylaminophenyl)- 17α-hydroxy-13α-methyl-4,9-gonadien-3-on.

11β-(4-Dimethylaminophenyl)- 17α-hydroxy-17β- (3-methoxy-1Z-propenyl)-13α-methyl-4,9-gonadien-3-on.

17β-Chlorethynyl-11β- (4-dimethylaminophenyl)- 17α-hydroxy-13α-methyl-4,9-gonadien-3-on.

17β-(2-Cyan)-ethyl-11β- (4-dimethylaminophenyl)- 17α-hydroxy-13α-methyl-4,9-gonadien-3-on.

17α, 21-Bis-acetoxy-11β-(4-dimethylaminophenyl)- 13α-methyl-18,19-dinor-4,9-pregnadien-3,20-dion.

11β-(4-Dimethylaminophenyl)-21-methoxy-13α-methyl-18,19-dinor-4,9-pregnadien-3,20-dion.

3-[11β-(4-Dimethylaminophenyl)- 17α-hydroxy-13α-methyl-3-oxo-4,9-gonadien-17β-yl]-propionsäurelacton.

Beispiel 1

a) Eine Lösung von 2,0 g 11β-(4-Dimethylaminophenyl)-3,3- (2,2-dimethyl-propan-1,3-dioxy)- 5α-hydroxy-9(10)-estren-17-on (F. 143–145 °C) in 300 ml absolutem Tetrahydrofuran (THF) wird 16 Minuten bei 25 °C mit einer Hg-Hochdrucklampe (Philips HPK 125, Tauchlampe, Quartzglasreaktor) bestrahlt. Anschliessend destilliert man das Lösungsmittel im Vakuum ab und chromatographiert den Rückstand über Aluminiumoxid (Merck, neutral, Stufe III) mit Hexan/Essigsäure. Man erhält 1,46 g 11β-(4-Dimethylaminophenyl)- 3,3-(2,2-dimethyl-propan-1,3-dioxy)- 5α-hydroxy-13α-methyl-9(10)-gonen-17-on als farbloses Öl.

b) Absolutes THF (248 ml) wird bei 5 °C durch 30-minütiges Einleiten mit Acetylen gesättigt. Anschliessend tropft man langsam 51 ml einer 15%igen Lösung von n-Butyllithium in Hexan hinzu und rührt weitere 15 Minuten unter Eiswasserkühlung. Eine Lösung von 2,7 g 11β-(4-Dimethylaminophenyl)- 3,3-(2,2-dimethyl-propan-1,3-dioxy) -5α-hydroxy-13α-methyl-9-gonen-17-on in 40 ml absolutem THF wird dann innerhalb von 15 Minuten zur Suspension des Lithiumacetylids getropft und weitere 2 Stunden bei Raumtemperatur gerührt. Zur Aufarbeitung giesst man in Eiswasser und extrahiert mit Essigester. Das so erhaltene Rohprodukt (2,85 g) wird ohne weitere Reinigung in die Folgestufe eingesetzt.

c) 2,8 g des unter b) erhaltenen Rohprodukts werden in 29 ml 70%iger wässriger Essigsäure suspendiert und 3 Stunden bei 50 °C gerührt. Nach dem Abkühlen verdünnt man mit ca. 100 ml Wasser und stellt durch Zugabe von konzentrierter wässriger $NH_3$-Lösung einen pH-Wert von 10,5 ein. Nach Extraktion mit Essigester erhält man ein öliges Isomerengemisch, das durch Säulenchromatographie an Kieselgel mit Hexan/Essigester getrennt wird. Man erhält in der Elutionsreihenfolge:

1. 530 mg 11β-(4-Dimethylaminophenyl)-17α-ethinyl-17β-hydroxy-13α-methyl-4,9-gonadien-3-on vom Schmelzpunkt 120–123 °C (Essigester/Diisopropylether) und

2. 1,33 g 11β-(4-Dimethylaminophenyl)-17β-ethinyl-17α-hydroxy-13α- methyl-4,9-gonadien-3-on vom Schmelzpunkt 201–204 °C (Essigester).

Analog b) und c) erhält man mit Methylacetylen anstelle von Acetylen:

1. 11β-(4-Dimethylaminophenyl)-17β-hydroxy-13α-methyl-17α-propinyl- 4,9-gonadien-3-on als Öl.

2. 11β-(4-Dimethylaminophenyl) -17α-hydroxy-13α-methyl-17β-propinyl-4,9-gonadien-3-on als Öl.

d) Eine Suspension von 1,02 g Quecksilberoxid (HgO, rot) in 20 ml Wasser wird nach Zugabe von 0,87 ml konzentrierter Schwefelsäure 30 Minuten bei 60 °C gerührt. 9 ml dieser Quecksilbersalz-Lösung werden zu einer Lösung von 3,25 g 11β-(4-Dimethylaminophenyl)-17β-ethinyl-17α-hydroxy-13α-methyl-4,9-gonadien-3-on in 32 ml Eisessig gegeben. Anschliessend rührt man 2 Stunden bei 60 °C. Zur Aufarbeitung giesst man die abgekühlte Reaktionslösung in Eiswasser, stellt durch Zugabe von konzentrierter wässriger $NH_3$-Lösung einen pH-Wert von 10,5 ein und extrahiert mit Ethylacetat. Das so erhaltene ölige Rohprodukt wird aus Methylenchlorid/Diisopropylether kristallisiert. Man erhält 2,37 g 11β- (4-Dimethylaminophenyl)-17α-hydroxy-13α-methyl-18,19-dinor-4,9-pregnadien-3,20-dion vom Schmelzpunkt 224-225 °C.

e) Eine Suspension von 2,3 g 11β-(4-Dimethylaminophenyl)- 17α-hydroxy-13α-methyl-18,19-dinor-4,9- pregnadien-3,20-dion in 58 ml Toluol wird nach Zugabe von 11,6 ml Acetanhydrid und 5,8 g 4-Dimethylaminopyridin 20 Stunden bei 25 °C gerührt. Danach giesst man in gesättigte $NaHCO_3$-Lösung und extrahiert mit Ethylacetat. Das Rohprodukt wird an 200 g Kieselgel mit Hexan/Ethylacetat chromatographiert. Nach Kristallisation der Hauptfraktion aus Hexan/Ethylacetat erhält man 1,71 g 17α-Acetoxy-11β-(4-dimethylaminophenyl)-13α-methyl- 18,19-dinor-4,9-pregnadien-3,20-dion vom Schmelzpunkt 194–195 °C.

Beispiel 2

a) Eine Lösung von 1,8 g 11β-(4-Diethylaminophenyl)3,3-(2,2-dimethyl-propan-1,3-dioxy) -5α-hydroxy-9-estren-17-on (F. 223–226 °C) in 300 ml THF wird unter den Bedingungen des Beispiels 1 a) 26 Minuten bestrahlt. Nach Chromatographie des Rohprodukts erhält man 1,58 g 11β-(4-Diethylaminophenyl)- 3,3- (2,2-dimethyl-propan-1,3-dioxy)- 5α-hydroxy-13α-methyl-9-gonen-17-on als farbloses Öl.

b) Aus 3,94 g 3-Tetrahydropyran-2'-yloxy-1-propin in 85 ml abs. THF und 23,1 ml einer 15%igen Lösung von n-Butyllithium in Hexan stellt man bei 0 °C die lithiumorganische Verbindung her. Anschliessend tropft man eine Lösung von 3,53 g des unter 2 a) beschriebenen Produkts in 71 ml abs. THF hinzu und rührt 4 Stunden bei Raumtemperatur. Die Reaktionslösung wird danach in Eiswasser gegossen und mit Essigester extrahiert. Das Rohprodukt (3,85 g) 11β-(4-Dimethylaminophenyl)- 3,3-(2,2-dimethyl-propan-1,3-dioxy)- 13α-methyl-17α-[3- (tetrahydropyran-2-yloxy) -1-propinyl]-9-gonen- 5α,-17β-diol und 11β-(4-Diethyöaminophenyl)-3,3-(2,2-dimethyl-propan-1,3-dioxy)-13α-methyl-17β- [3-(tetrahydropyran-2-yloxy)-1-propinyl]-9-gonen-5α, 17α-diol wird ohne weitere Reinigung zur Hydrierung eingesetzt.

c) 3,85 g des unter 2 b) erhaltenen Rohprodukts werden in 95 ml Ethanol nach Zusatz von 400 mg 10%iger Palladiumkohle bei Raumtemperatur und Normaldruck hydriert. Nach Aufnahme von 191 ml Wasserstoff filtriert man vom Katalysator ab und engt ein.

d) Das unter 2 c) erhaltene Hydrierungsrohprodukt (3,85 g) wird in 30 ml 70%iger Essigsäure 2 Stunden bei 60 °C gerührt. Nach dem Abkühlen arbeitet man wie unter 1 c) auf und chromatographiert das erhaltene Isomerengemisch. In der Elutionsreihenfolge erhält man:

1. 410 mg 11β-(4-Diethylaminophenyl)- 17α-(3-hydroxy-propyl) -17β-hydroxy-13α-methyl-4,9-gonadien-3-on als gelbliches Öl.

UV (Methanol): $\varepsilon_{266}$ = 19080, $\varepsilon_{309}$ = 19110.

2. 1,39 g 11β-(4-Diethylaminophenyl)- 17α-(3-hydroxypropyl)- 17α-hydroxy-13α-methyl-4,9-gonadien-3-on als festen Schaum.

Analog b) bis d) erhält man bei der Verwendung von 11β-(4-Dimethylaminophenyl)-3,3-(2,2-dimethyl-propan-1,3-dioxy) -5α-hydroxy-13α-methyl-9-gonen-17-on als Ausgangsmaterial:

1. 11β-(4-Dimethylaminophenyl)-17β-hydroxy-17α- (3-hydroxypropyl)-13α-methyl-4,9-gonadien-3-on als Öl.

2. 11β-(4-Dimethylaminophenyl) -17α-hydroxy-17β- (3-hydroxypropyl)- 13α-methyl-4,9-gonadien-3-on als Öl.

Beispiel 3

a) Eine Lösung von 1,75 g 3,3-(2,2-Dimethyl-propan-1,3-dioxy)- 5α-hydroxy-11β- (3-methoxyphenyl)- 9-estren-17-on in 290 ml Dioxan wird 19 Minuten unter den Bedingungen des Beispiels 1 a) bestrahlt. Nach Chromatographie erhält man 1,45 g 3,3-(2,2-Dimethyl-propan-1,3-dioxy)- 5α-hydroxy-11β- (4-methoxyphenyl)- 13α-methyl-9-gonen-17-on als farbloses Öl.

b) Zu einer Suspension von Lithiumacetylid, hergestellt aus einer gesättigten Lösung von Acetylen in 450 ml THF und 130 ml einer 15%igen Lösung von n-Butyllithium in Hexan, tropft man eine Lösung von 8,2 g 3,3-(2,2-Dimethyl-propan-1,3-dioxy) -5α-hydroxy-11β-(4-methoxyphenyl)- 13α-methyl-9-gonen-17-on in 130 ml THF. Anschliessend rührt man 3 Stunden bei Raumtemperatur, giesst danach die Reaktionslösung in ca. 3 l Eiswasser und extrahiert mit Ethylacetat. Das Rohprodukt wird an Aluminiumoxid mit Hexan/Ethylacetat chromatographiert. In der Elutionsreihenfolge erhält man:

1. 1,8 g 3,3-(2,2-Dimethyl-propan-1,3-dioxy) -17α-ethinyl-11β- (4-methoxyphenyl)- 13α-methyl-9-gonen-5α,17β-diol als farbloses Öl.

2. 5,1 g 3,3-(2,2-Dimethyl-propan-1,3-dioxy)-17β- ethinyl-11β- (4-methoxyphenyl)-13α-methyl- 9-gonen-5α,17α-diol als festen Schaum.

c) Eine Lösung von 3,28 g 3,3-(2,2-Dimethyl-propan-1,3-dioxy)17β-ethinyl-11β-(4-methoxyphenyl)- 13α-methyl-9-gonen-5α,17α-diol in 33 ml 70%iger wässriger Essigsäure wird 30 Minuten bei 60 °C gerührt. Nach dem Abkühlen giesst man in Eiswasser, extrahiert mit Methylenchlorid, wäscht die $MeCl_2$-Extrakte mit gesättigter $NaHCO_3$-Lösung und engt ein. Kristallisation des Rohprodukts aus Ethylacetat ergibt 2,0 g 17β-Ethinyl- 17α-hydroxy-11β- (4-methoxyphenyl)- 13α-methyl- 4,9-gonadien-3-on vom Schmelzpunkt 186–187 °C.

Beispiel 4

20-minütige Bestrahlung von 1,84 g 11β-(4-Dimethylaminophenyl)- 3,3-(2,2-dimethyl-propan-1,3-dioxy)- 5α-hydroxy-18-methyl-9-estren-17-on in 280 ml THF unter den Bedingungen des Beispiels 1 a) führt zu 1,36 g 11β-(4-Dimethylaminophenyl)-3,3- (2,2-dimethyl-propan-1,3-dioxy)- 13α-ethyl-5α-hydroxy-9-gonen-17-on als Schaum.

6,1 g 11β- (4-Dimethylaminophenyl)- 3,3-(2,2-dimethylpropan-1,3-dioxy) -13α-ethyl-5α-hydroxy-9-gonen-17-on werden unter den Bedingungen des Beispiels 1 b) mit Lithiumacetylid umgesetzt und das so erhaltene Rohprodukt unter den Bedingungen des Beispiels 1 c) mit Essigsäure gespalten. Nach Chromatographie und Kristallisation aus Ethylacetat/Diisopropylether erhält man 3,2 g 11β-(4-Dimethylaminophenyl)-17β-ethinyl-13α-ethyl-17α- hydroxy-4,9-gonadien-3-on vom Schmelzpunkt 197–198 °C,

$[\alpha]_D^{25}$ + 450,4° ($CHCl_3$, c=0,505).

Durch Quecksilbersalz-katalysierte Hydratisierung analog Beispiel 1 d) und anschliessende Acetylierung analog Beispiel 1 e) erhält man aus 1,3 g 11β-(4-Dimethylaminophenyl)- 17β-ethinyl-13α-ethyl-17α-hydroxy- 4,9-gonadien-3-on nach chromatographischer Reinigung 720 mg 17α- Acetoxy- 11β- (4-dimethylaminophenyl)-13α-ethyl- 18,19-dinor- 4,9-pregnadien-3,20-dion als festen Schaum.

$[\alpha]_D^{25}$ + 290,8° ($CHCl_3$, c=0,515).

Beispiel 5

a) Durch Umsetzung von 7,3 g 11β-(4-Dimethylaminophenyl)-3,3- (2,2-dimethyl-propan-1,3-oxy)- 5α-hydroxy-13α-methyl-9-gonen-17-on mit 10,7 g 3-Tetrahydropyran-2′-yloxy-1-propin unter den Bedingungen des Beispiels 2 b) erhält man nach Chromatographie des Rohprodukts an Aluminiumoxid mit Hexan/Ethylacetat 4,83 g 11β-(4-Dimethylaminophenyl)- 3,3-(2,2-dimethyl-propan-1,3-dioxy)- 13α-methyl-17β-[3-(tetrahydropyran-2-yloxy)- 1-propinyl]-9-gonen-5α,17α-diol als gelblichen Schaum.

b) Eine Lösung von 2,2 g des unter a) erhaltenen Addukts in 67 ml Ethanol und 0,56 ml Triethylamin wird nach Zusatz von 210 mg Palladium auf Bariumsulfat (10%) bei Raumtemperatur und Normaldruck hydriert. Nach Aufnahme von 83,5 ml Wasserstoff wird vom Katalysator abfiltriert und eingeengt. Das so erhaltene Hydrierungsprohprodukt wird mit 14 ml 70%iger Essigsäure unter den Bedingungen des Beispiels 1 c) gespalten. Nach Kristallisation aus Ethylacetat/Diisopropylether erhält man 1,1 g 11β-(4-Dimethylaminophenyl)17α-hydroxy-17β- (3-hydroxy-1(Z)-propenyl)-13α-methyl-4,9- gonadien-3-on vom Schmelzpunkt 133–135 °C.

Beispiel 6

Herstellung von 11β-(4-Dimethylaminophenyl)- 17β-ethinyl-16β-ethyl-17α-hydroxy- 13α-methyl-4,9-gonadien-3-on

a) Eine Suspension aus 29,3 g 3,3-(2,2-Dimethyl-propan-1,3-dioxy)-5(10),9(11)-estradien-17-on und 28,6 g Bis-dimethylamino-tert.-butoxymethan wird unter Argon 60 Minuten bei 160 °C gerührt. Nach dem Abkühlen verreibt man das Rohprodukt mit ca. 50 ml Ethylacetat, filtriert und kristallisiert den Filtrierrückstand aus Ethylacetat um. Auf diese Weise erhält man 27,6 g 16-Dimethylaminomethylen-3,3-(2,2-dimethyl-propan-1,3-dioxy) -5-(10),9(11)-estradien-17-on vom Schmelzpunkt 208–211 °C.

b) Eine Lösung von 14,4 g 16-Dimethylaminomethylen-3,3- (2,2-dimethyl-propan-1,3-dioxy)- 5(10),9(11)-estradien-17-on in 220 ml Toluol wird unter Eiswasserkühlung tropfenweise mit 85 ml einer 5%igen Lösung von Methyllithium in Diethylether versetzt. Nach Zugabe rührt man 15 Minuten bei +5 bis +10 °C, zersetzt überschüssiges Reagenz durch vorsichtige Zugabe von ca. 20 ml Wasser, giesst die Reaktionslösung anschliessend in ca. 3 l Eiswasser und extrahiert mit Methylenchlorid. Das Rohprodukt wird an neutralem Aluminiumoxid mit Hexan/Ethylacetat chromatisiert. Nach Kristallisation der Hauptfraktion aus Ethylacetat erhält man 13,0 g 3,3-(2,2-Dimethyl-propan-1,3-dioxy) -16(E)-ethyliden-5(10),9(11)-estradien-17-on vom Schmelzpunkt 121–123 °C.

c) Zu einer Lösung von 9,4 g 3,3-(2,2-Dimethyl-propan-1,3-dioxy)-16(E)-ethyliden- 5(10), 9(11)- estradien-17-on in 43 ml Methylenchlorid, 0,34 ml Hexachloraceton und 0,01 ml Pyridin tropft man unter Eiswasserkühlung 4,3 ml 30%igen Wasserstoffperoxid und rührt anschliessend 16 Stunden bei 25 °C. Zur Aufarbeitung verdünnt man die Reaktionslösung mit ca. 100 ml Methylenchlorid, wäscht nacheinander mit 5%iger $Na_2S_2O_3$-Lösung und Wasser, trocknet die Methylenchlorid-Phase über $Na_2SO_4$ und engt ein. Das so erhaltene 5,10-Epoxidgemisch wird an $Al_2O_3$, neutral, Stufe III, mit Hexan/Ethylacetat chromatographiert. Man erhält 4,7 g 3,3-(2,2-Dimethylpropan-1,3-dioxy)- 5α,10α-epoxy- 16(E)-ethyliden- 9(11)-estren- 17-on vom Schmelzpunkt 139–141 °C (Ethylacetat/Diisopropylether).

d) Eine Lösung von 8,2 g 3,3-(2,2-Dimethyl-propan- 1,3-dioxy)5α,10α-epoxy- 16(E)-ethyliden-9(11) -estren-17-on in 400 ml Ethanol wird nach Zusatz von 930 mg Palladiumkohle (10%) bei Raumtemperatur und Normaldruck hydriert. Nach Aufnahme von 510 ml Wasserstoff wird vom Katalysator abfiltriert und im Vakuum eingeengt. Man erhält 7,7 g 3,3-(2,2-Dimethyl- propan-1,3-dioxy)- 5α,10α-epoxy-16β-ethyl-9(11)-estren-17-on als farbloses Ol.

e) aus 1,4 g Magnesium, 0,05 ml Methyljodid und 17,9 g 4-Brom-N,N-dimethylanilin in 150 ml abs. THF wird die magnesiumorganische Verbindung hergestellt. Nach Zusatz von 344 g CuCl rührt man 15 Minuten bei 0 °C und gibt dann tropfenweise eine Lösung von 7,7 g des unter d) erhaltenen Produkts in 70 ml abs. THF hinzu. Danach wird 3,5 Stunden bei Raumtemperatur gerührt. Zur Aufarbeitung giesst man die Reaktionslösung in ein Gemisch aus Eiswasser und $NH_3$-Lösung und extrahiert mit Ethylacetat. Nach Chromatographie des Rohprodukts an Aluminiumoxid mit Hexan/Ethylacetat und Kristallisation der Hauptfraktion aus Diisopropylether/Ethylacetat erhält man 6,5 g 11β-(4-Dimethylaminophenyl)- 3,3-(2,2-dimethylpropan-1,3-dioxy)-16β-ethyl- 5α-hydroxy- 9(10)-estren-17-on vom Schmelzpunkt 180–181 °C.

f) Eine Lösung von 4,0 g des unter e) erhaltenen Produkts in 600 ml Dioxan wird unter den Bedingungen des Beispiels 1 a) bestrahlt. Nach Kristallisation des Bestrahlungsrohprodukts aus Diisopropylether erhält man 1,74 g 11β-(4-Dimethylaminophenyl)- 3,3-(2,2-dimethyl-propan- 1,3-dioxy)- 16β-ethyl-5α-hydroxy- 13α-methyl-9(10)-gonen-17-on vom Schmelzpunkt 192–194 °C.

g) Unter den Bedingungen des Beispiels 1 b) werden 1,4 g des unter f) erhaltenen Produkts mit Lithiumacetylid umgesetzt. Nach Kristallisation des Rohprodukts aus Ethylacetat/Diisopropylether erhält man 960 mg 11β-(4-Dimethylaminophenyl)- 3,3- (2,2-dimethyl- propan- 1,3-dioxy)-17β- ethinyl-16β-ethyl-13α-methyl-9(10)-gonen-5α,17α- diol vom Schmelzpunkt 132–134 °C.

h) Unter den Bedingungen des Beispiels 1 c) setzt man 760 mg des unter g) erhaltenen Produkts mit 8 ml 70%iger Essigsäure um. Kristallisation des Rohprodukts aus Hexan/Diethylether ergibt 460 mg 11β-(4-Dimethylaminophenyl)-17β-ethinyl-16β- ethyl-17α-hydroxy-13α-methyl- 4,9-gonadien-3-on vom Schmelzpunkt 195–197 °C.

Beispiel 7

Herstellung von 17β-Cyanmethyl-11β- (4-dimethylaminophenyl)- 17α-hydroxy-13α-methyl-4,9-gonadien-3-on

a) Eine Suspension von 5,0 g 11β-(4-Dimethylaminophenyl)- 3,3-(2,2-dimethyl-propan-1,3-dioxy)- 5α-hydroxy-13α-methyl-9(10)-gonen-17-on und 4,74 g Trimethylsulfoniumiodid in 60 ml Dimethylformamid wird unter Eiswasserkühlung portionsweise mit 2,63 g Kalium-tert.-butylat versetzt. Man rührt 4 Stunden bei 25 °C, giesst anschliessend in Eiswasser und extrahiert

mit Ethylacetat. Nach Entfernen des Lösungsmittels kristallisiert man das zunächst ölige Rohprodukt aus Ethylacetat/Diisopropylether und erhält auf diese Weise 4,2 g 11β-(4-Dimethylaminophenyl)- 3,3-(2,2-dimethyl-propan-1,3-dioxy) -5α-hydroxy-13α-methyl-9(10)-gonen-17α-spiro- 1',2'-oxiran vom Schmelzpunkt 234–236 °C.

b) 2,0 g des unter a) erhaltenen Spiro-oxirans werden in 84 ml Ethanol gelöst und nach Zusatz von 4,6 g Kaliumcyanid 4 Stunden unter Rückfluss erhitzt. Die abgekühlte Lösung wird in gesättigte $NaHCO_3$-Lösung gegossen und mit Ethylacetat extrahiert. Das nach dem Einengen erhaltene Rohprodukt wirdohne weitere Reinigung in 26 ml 70%ige Essigsäure aufgenommen und 60 Minuten bei 60 °C gerührt. Nach dem Abkühlen giesst man in Eiswasser, stellt durch Zugabe von $NH_3$-Lösung einen pH-Wert von 10,5 ein und extrahiert mit Methylenchlorid. Nach Chromatographie des Rohprodukts an Kieselgel mit Hexan/Aceton und Kristallisation der Hauptfraktion aus Ethanol erhält man 1,4 g 17β-Cyanmethyl-11β-(4-dimethylaminophenyl)- 17α-hydroxy-13α-methyl-4,9- gonadien-3-on vom Schmelzpunkt 135–137 °C.

## Patentansprüche für die Vertragsstaaten BE, CH, DE, FR, GB, IT, LU, NL, SE

1. 13α-Alkylgonane der allgemeinen Formel I

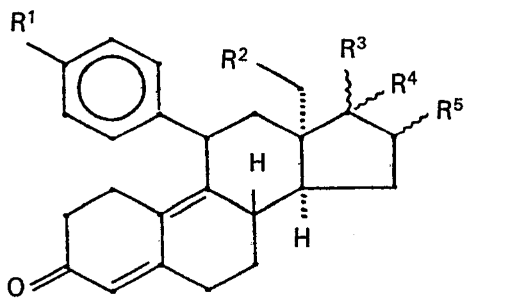

(I),

worin

$R^1$ $-N<^{R^I}_{R^{II}}$ mit $R^I$ und $R^{II}$ in der Bedeutung von Wasserstoff oder Alkyl mit 1 bis 4 Kohlenstoffatomen oder $R^I$ und $R^{II}$ unter Einschluss von N in der Bedeutung eines gesättigten 5- oder 6-gliedrigen Ringes, wobei im Ring ausser N noch ein weiteres Heteroatom wie O, N, S enthalten sein kann, sowie die entsprechenden tertiären N-Oxide und die Säureadditionssalze,

$-OR^{III}$ mit $R^{III}$ in der Bedeutung von Methyl, Ethyl, Propyl, Methoxyphenyl, Allyl oder β-Dimethylaminoethyl,

$R^2$ ein Wasserstoffatom, eine Methyl- oder Ethylgruppe,

$R^3$ $-(CH_2)_n-CH_3$ mit n=0–4,

$-(CH_2)_n-CH_2-O(S)R^{IV}$ mit n=0–4 und $R^{IV}$ in der Bedeutung von Wasserstoff oder Alkyl mit 1 bis 4 Kohlenstoffatomen,

$-CH=CH-(CH_2)_n-OR^V$ mit n=1–4 und $R^V$ in der Bedeutung von Wasserstoff, Alkyl oder Alkanoyl mit jeweils 1 bis 4 Kohlenstoffatomen,

$-C\equiv C-X$ mit X in der Bedeutung von Wasserstoff, Alkyl mit 1 bis 4 Kohlenstoffatomen oder Halogen,

$-(CH_2)_n-CH_2CN$ mit n=0–3 oder

$-\overset{O}{\overset{\|}{C}}-CH_2Y$ mit Y in der Bedeutung von Wasserstoff oder $OR^V$ mit $R^V$ in der oben angegebenen Bedeutung,

$R^4$ Hydroxy, Alkyloxy oder Alkanoyloxy mit jeweils 1 bis 4 Kohlenstoffatomen oder

$R^3/R^4$ 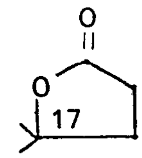 wobei

$R^3$ in α-Stellung und $R^4$ in β-Stellung oder $R^3$ in β-Stellung und $R^4$ in α-Stellung zum Steroidgerüst stehen, und

$R^5$ ein Wasserstoffatom oder eine α- oder β-ständige Alkylgruppe mit 1 bis 4 Kohlenstoffatomen bedeuten.

2. 11β-(4-Dimethylaminophenyl)- 17α-ethinyl- 17β-hydroxy- 13α-methyl-4,9- gonadien-3-on und
11β-(4-Dimethylaminophenyl)- 17β-ethinyl-17α-hydroxy-13α-methyl-4,9-gonadien-3-on.

3. 11β- (4-Dimethylaminophenyl)-17β-hydroxy-13α-methyl- 17α-propinyl-4,9-gonadien-3-on und
11β-(4-Dimethylaminophenyl)- 17α-hydroxy-13α-methyl-17β- propinyl-4,9-gonadien-3-on.

4. 11β-(Dimethylaminophenyl)- 17α-hydroxy-13α-methyl-18,19-dinor-4,9-pregnadien-3,20-dion und
17α- Acetoxy- 11β- (4-dimethylaminophenyl)-13α-methyl-18,19-dinor-4,9-pregnadien-3,20-dion.

5. 11β-(4-Diethylaminophenyl)17α-(3-hydroxypropyl)-17β-hydroxy- 13α-methyl-4,9-gonadien-3-on und
11β- (4-Diethylaminophenyl)- 17β- (3-hydroxypropyl)- 17α-hydroxy-13α-methyl-4,9-gonadien-3-on.

6. 11β-(4-Dimethylaminophenyl)-17β-hydroxy-17α- (3-hydroxypropyl)- 13α-methyl-4,9-gonadien-3-on und
11β- (4-Dimethylaminophenyl)- 17α- hydroxy-17β- (3-hydroxypropyl)- 13α-methyl-4,9-gondien-3-on.

7. 17β- Ethinyl- 17α-hydroxy-11β- (4-methoxyphenyl)- 13α-methyl-4,9-gonadien-3-on.

8. 11β-(4-Dimethylaminophenyl)- 17β-ethinyl-13α-ethyl-17α-hydroxy-4,9-gonadien-3-on und
17α-Acetoxy-11β-(4-dimethylaminophenyl)-13α-ethyl-18,19-dinor-4,9-pregnadien-3,20-dion.

9. 11β-(4-Dimethylaminophenyl)-17α-hydroxy- 17β-(3-hydroxy- 1(Z)-propenyl)- 13α-methyl-4,9-gonadien-3-on.

10. 11β-(4-Dimethylaminophenyl)- 17β-ethinyl- 16β- ethyl-17α-hydroxy- 13α-methyl-4,9-gonadien- 3-on.

11. 17β-Cyanmethyl-11β- (4-dimethylamino-phenyl)- 17α-hydroxy-13α-methyl- 4,9-gona-dien-3-on.

12. Pharmazeutische Präparate, gekennzeichnet durch den Gehalt an Verbindungen gemäss Anspruch 1 bis 11.

13. Verfahren zur Herstellung von 13α-Alkylgonanen der allgemeinen Formel I

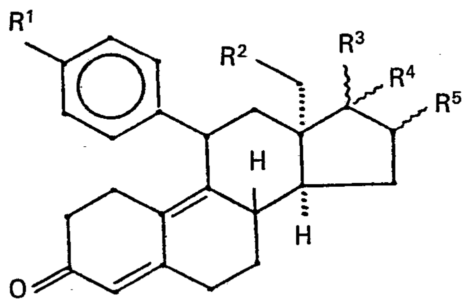

(I),

worin $R^1$, $R^2$, $R^3$, $R^4$ und $R^5$ die in Anspruch 1 angegebene Bedeutung haben, dadurch gekennzeichnet, dass man eine Verbindung der allgemeinen Formel II

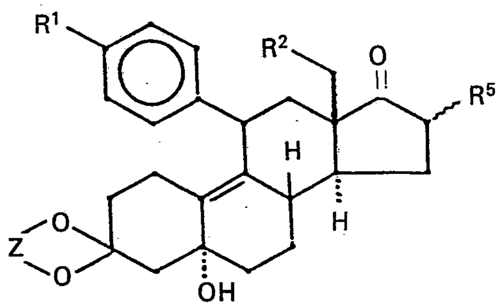

(II),

worin $R^1$, $R^2$ und $R^5$ die in Formel I angegebene Bedeutung haben und Z eine Ethylen- oder 2,2-Dimethylpropylengruppe darstellt, mit ultraviolettem Licht bestrahlt und das durch UV-Bestrahlung erhaltene 13-Episteroid der allgemeinen Formel III

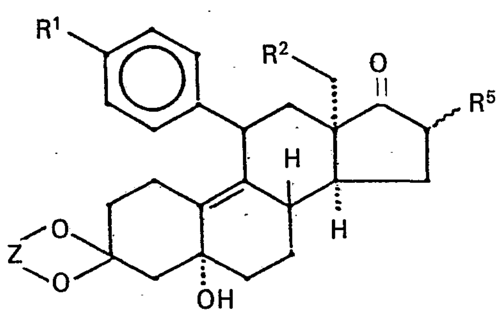

(III),

worin $R^1$, $R^2$, $R^5$ und Z die in der Formel II angegebene Bedeutung haben, nach an sich bekannten Methoden durch nucleophile Addition an das 17-Keton, Spaltung des 3-Ketalschutzes und Wasserabspaltung aus 4,5-Stellung in die Verbindungen der allgemeinen Formel I überführt.

14. 13-Episteroide der allgemeinen Formel III

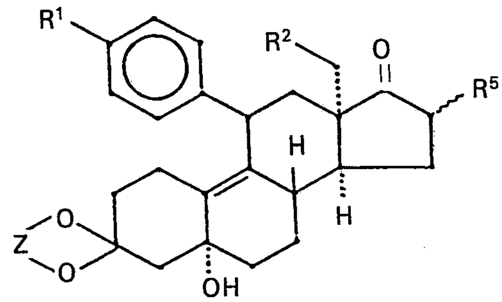

(III),

worin

$R^1$ $-N<^{R^I}_{R^{II}}$ mit $R^I$ und $R^{II}$ in der Bedeutung von Wasserstoff oder Alkyl mit 1 bis 4 Kohlenstoffatomen oder $R^I$ und $R^{II}$ unter Einschluss von N in der Bedeutung eines gesättigten 5- oder 6-gliedrigen Ringes, wobei im Ring ausser N noch ein weiteres Heteroatom wie O, N, S enthalten sein kann, sowie die entsprechenden tertiären N-Oxide und die Säureadditionssalze,

$-OR^{III}$ mit $R^{III}$ in der Bedeutung von Methyl, Ethyl, Propyl, Methoxyphenyl, Allyl oder β-Dimethylaminoethyl,

$R^2$ ein Wasserstoffatom, eine Methyl- oder Ethylgruppe,

$R^5$ ein Wasserstoffatom oder eine α- oder β-ständige Alkylgruppe mit 1 bis 4 Kohlenstoffatomen und

Z eine Ethylen- oder 2,2-Dimethylpropylengruppe bedeuten.

**Patentanspruch für den Vertragsstaat AT**

Verfahren zur Herstellung von 13α-Alkylgonanen der allgemeinen Formel I

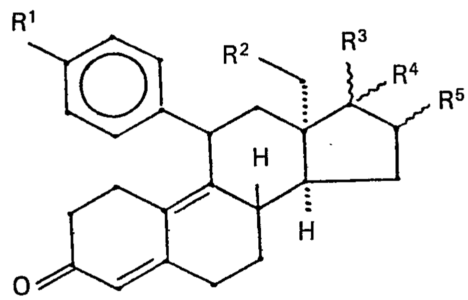

(I),

worin

$R^1$ $-N<^{R^I}_{R^{II}}$ mit $R^I$ und $R^{II}$ in der Bedeutung von Wasserstoff oder Alkyl mit 1 bis 4 Kohlenstoffatomen oder $R^I$ und $R^{II}$ unter Einschluss von N in der Bedeutung eines gesättigten 5- oder 6-gliedrigen Ringes, wobei im Ring ausser N noch ein weiteres Heteroatom wie O, N, S enthalten sein kann, sowie die entsprechenden tertiären N-Oxide und die Säureadditionssalze,

$-OR^{III}$ mit $R^{III}$ in der Bedeutung von Methyl, Ethyl, Propyl, Methoxyphenyl, Allyl oder β-Dimethylaminoethyl,

$R^2$ ein Wasserstoffatom, eine Methyl- oder Ethylgruppe,

$R^3$ $-(CH_2)_n-CH_3$ mit n=0–4,

$-(CH_2)_n-CH_2-O(S)R^{IV}$ mit n=0–4 und $R^{IV}$ in der Bedeutung von Wasserstoff oder Alkyl mit 1 bis 4 Kohlenstoffatomen,

$-CH=CH-(CH_2)_n-OR^V$ mit n=1–4 und $R^V$ in der Bedeutung von Wasserstoff, Alkyl oder Alkanoyl mit jeweils 1 bis 4 Kohlenstoffatomen,

$-C\equiv C-X$ mit X in der Bedeutung von Wasserstoff, Alkyl mit 1 bis 4 Kohlenstoffatomen oder Halogen,

$-(CH_2)_n-CH_2CN$ mit n=0–3 oder

$-\overset{O}{\overset{\|}{C}}-CH_2Y$ mit Y in der Bedeutung von Wasserstoff oder $OR^V$ mit $R^V$ in der oben angegebenen Bedeutung,

$R^4$ Hydroxy, Alkyloxy oder Alkanoyloxy mit jeweils 1 bis 4 Kohlenstoffatomen oder

$R^3/R^4$ 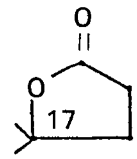

wobei $R^3$ in α-Stellung und $R^4$ in β-Stellung oder $R^3$ in β-Stellung und $R^4$ in α-Stellung zum Steroidgerüst stehen, und

$R^5$ ein Wasserstoffatom oder eine α-oder β-ständige Alkylgruppe mit 1 bis 4 Kohlenstoffatomen bedeuten, dadurch gekennzeichnet, dass man eine Verbindung der allgemeinen Formel II

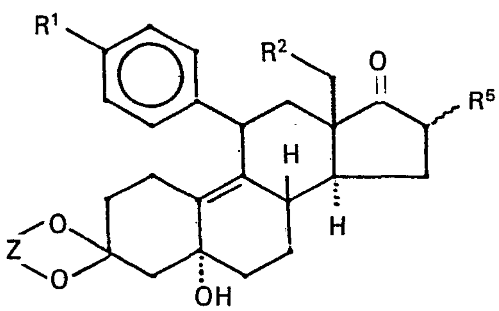

(II),

worin $R^1$, $R^2$ und $R^5$ die in Formel I angegebene Bedeutung haben und Z eine Ethylen- oder 2,2-Dimethylpropylengruppe darstellt, mit ultraviolettem Licht bestrahlt und das durch UV-Bestrahlung erhaltene 13-Episteroid der allgemeinen Formel III

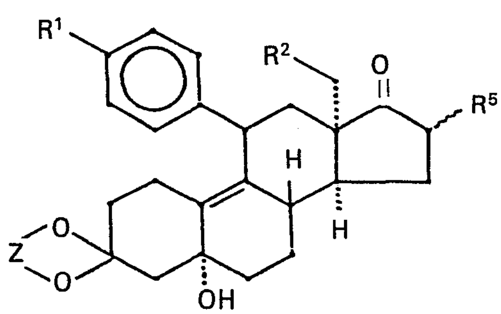

(III),

worin $R^1$, $R^2$, $R^5$ und Z die in der Formel II angegebene Bedeutung haben, nach an sich bekannten Methoden durch nucleophile Addition an das 17-Keton, Spaltung des 3-Ketalschutzes und Wasserabspaltung aus 4,5-Stellung in die Verbindungen der allgemeinen Formel I überführt.

**Revendications pour les Etats contractants BE, CH, LI, DE, FR, GB, IT, LU, NL, SE**

1. Alkyl-13α gonanes qui répondent à la formule générale I:

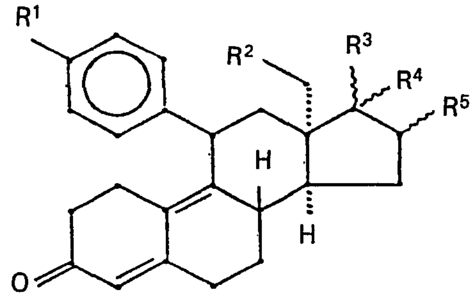

(I)

dans laquelle

$R^1$ représente un radical $-N<^{R^I}_{R^{II}}$ dont les symboles $R^I$ et $R^{II}$ représentent chacun l'hydrogène ou un alkyle contenant de 1 à 4 atomes de carbone ou forment ensemble et avec l'atome N un cycle saturé à 5 ou 6 maillons, ce cycle pouvant contenir en outre, en plus de N, un hétéroatome supplémentaire, tel que O, N ou S, ou représente un radical N-oxyde tertiaire correspondant ou un sel d'addition d'acide correspondant, et $R^1$ peut également représenter un radical $-OR^{III}$ dans lequel $R^{III}$ désigne un radical méthyle, éthyle, propyle, méthoxyphényle, allyle ou diméthylamino-2-éthyle,

$R^2$ représente un atome d'hydrogène ou un radical méthyle ou éthyle,

$R^3$ représente:

– un radical $-(CH_2)_n-CH_3$ dans lequel n désigne un nombre de 0 à 4,

– un radical $-(CH_2)_n-CH_2-O(S)R^{IV}$ dans lequel n désigne un nombre de 0 à 4 et $R^{IV}$ représente l'hydrogène ou un alkyle contenant de 1 à 4 atomes de carbone,

– un radical $-CH=CH-(CH_2)_n-OR^V$ dans lequel n désigne un nombre de 1 à 4 et $R^V$ représente l'hydrogène, ou un alkyle ou un alcanoyle contenant chacun de 1 à 4 atomes de carbone,

– un radical $-C\equiv C-X$ dans lequel X représente l'hydrogène, un alkyle contenant de 1 à 4 atomes de carbone ou un halogène,

– un radical $-(CH_2)_n-CH_2CN$ dans lequel n désigne un nombre de 0 à 3 ou

– un radical $-\overset{O}{\overset{\|}{C}}-CH_2Y$ dans lequel Y représente l'hydrogène ou un radical $OR^V$ dont le symbole $R^V$ a la signification qui lui a été donnée ci-dessus,

$R^4$ représente un hydroxy, ou un alcoxy ou un alcanoyloxy contenant chacun de 1 à 4 atomes de

carbone, ou encore $R^3$ et $R^4$ forment ensemble un radical:

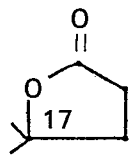

le substituant $R^3$ ayant la configuration α et le substituant $R^4$ la configuration β, ou inversement, sur le squelette stéroïdique, et

$R^5$ représente un atome d'hydrogène ou un radical alkyle qui contient de 1 à 4 atomes de carbone et qui a la configuration α ou la configuration β.

2. (Diméthylamino-4 phényl)-11β éthynyl-17α hydroxy-17β-méthyl-13α gonadiène-4,9 one-3 et
(diméthylamino-4 phényl)-11β éthynyl-17β hydroxy-17α méthyl-13α gonadiène-4,9 one-3.

3. (Diméthylamino-4 phényl)-11β hydroxy-17β méthyl-13α propynyl-17α gonadiène-4,9 one-3 et
(diméthylamino-4-phényl)-11β hydroxy-17α méthyl-13α propynyl-17β gonadiène-4,9 one-3.

4. (Dimethylamino-4 phényl)-11β hydroxy-17α méthyl-13α dinor-18,19 prégnadiène-4,9 dione-3,20 et
acétoxy-17α (diméthylamino-4 phényl)-11β-méthyl-13α dinor-18,19 prégnadiène-4,9 dione-3,20.

5. (Diéthylamino-4 phényl)-11β (hydroxy-3 propyl)-17α hydroxy-17β méthyl-13α gonadiène-4,9 one -3 et
(diéthylamino-4 phényl)-11β (hydroxy-3 propyl)-17β hydroxy-17α méthyl-13α gonadiène-4,9 one-3.

6. (Diméthylamino-4 phényl)-11β hydroxy-17β (hydroxy-3 propyl)-17α méthyl-13α gonadiène-4,9 one-3 et
(diméthylamino-4 phényl)-11β hydroxy-17α (hydroxy-3 propyl)-17β méthyl-13α gonadiène-4,9 one-3.

7. Ethynyl-17β hydroxy-17α (méthoxy-4 phényl)-11β méthyl-13α gonadiène-4,9 one-3.

8. (Diméthylamino-4 phényl)-11β éthynyl-17β éthyl-13α hydroxy-17α gonadiène-4,9 one-3 et acétoxy-17α (diméthylamino-4 phényl)-11β éthyl-13α dinor-18,19 prégnadiène-4,9 dione-3,20.

9. (Diméthylamino-4 phényl)-11β hydroxy-17α (hydroxy-3 propène-1(Z) yl)-17β méthyl-13α gonadiène-4,9 one-3.

10. (Diméthylamino-4 phényl)-11β éthynyl-17β éthyl-16β hydroxy-17α méthyl-13α gonadiène-4,9 one-3.

11. Cyanométhyl-17β (diméthylamino-4 phényl)-11β hydroxy-17α méthyl-13α gonadiène-4,9 one-3.

12. Compositions pharmaceutiques caractérisées en ce qu'elles contiennent des composés selon l'une quelconque des revendications 1 à 11.

13. Procédé de préparation d'alkyl-13α gonanes répondant à la formule générale I:

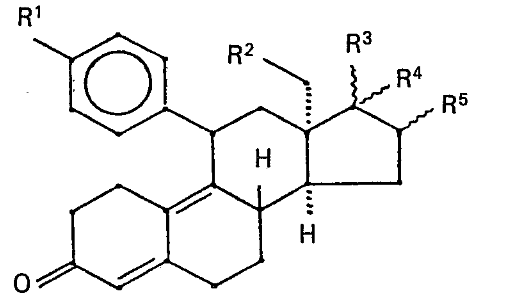

(I)

dans laquelle $R^1$, $R^2$, $R^3$, $R^4$ et $R^5$ ont les significations données à la revendication 1, procédé caractérisé en ce qu'on irradie par un rayonnement ultra-violet un composé répondant à la formule générale II:

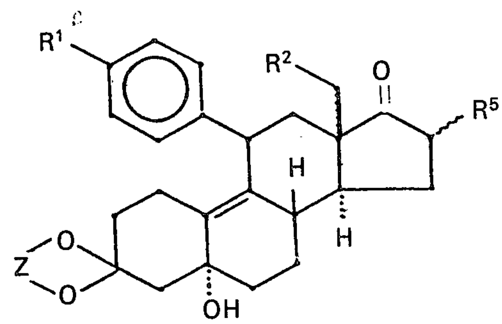

(II)

dans laquelle $R^1$, $R^2$ et $R^5$ ont les significations données à propos de la formule I et Z représente un radical éthylène ou diméthyl-2,2 propylène, et on transforme l'épi-13 stéroïde qui s'est formé sous l'action du rayonnement ultra-vilet et qui répond à la formule générale III:

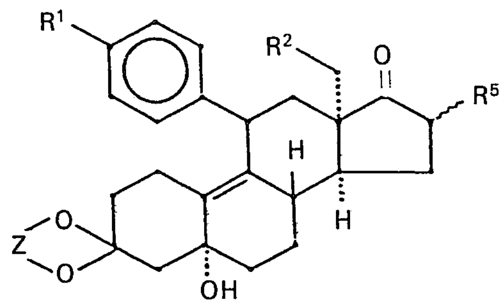

(III)

dans laquelle $R^1$, $R^2$, $R^5$ et Z ont les significations données à propos de la formule II, en appliquant des méthodes connues, par addition nucléophile sur le radical cétonique en 17, coupure du radical acétal protecteur en 3 et/ou enlèvement d'eau en position 4,5, en composés de formule générale I.

14. Epi-13 stéroïdes répondant à la formule générale III:

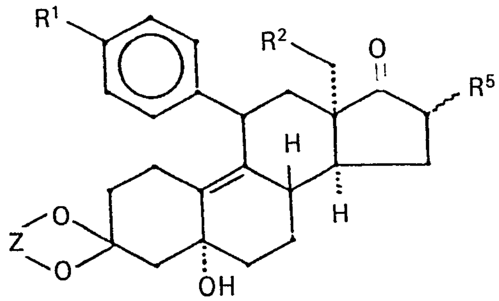

(III)

dans laquelle

$R^1$ représente un radical $-N{<}{R^{I} \atop R^{II}}$ dont les symboles $R^I$ et $R^{II}$ représentant chacun l'hydrogène ou un alkyle contenant de 1 à 4 atomes de carbone ou forment ensemble et avec l'atome N un cycle saturé à 5 ou 6 maillons, ce cycle pouvant contenir en outre, en plus de N, un hétéroatome supplémentaire, tel que O, N ou S, ou représente un radical N-oxyde tertiaire correspondant ou un sel d'addition d'acide correspondant, et $R^1$ peut également représenter un radical $-OR^{III}$ dans lequel $R^{III}$ désigne un radical méthyle, éthyle, propyle, méthoxyphényle, allyle ou diméthylamino-2 éthyle,

$R^2$ représente un atome d'hydrogène, un radical méthyle ou un radical éthyle,

$R^5$ représente un atome d'hydrogène ou un radical alkyle contenant de 1 à 4 atomes de carbone et ayant la configuration α ou la configuration β et

Z représente un radical éthylène ou un radical diméthyl-2,2 propylène.

**Revendications pour l'Etat contractant: AT**

Procédé de préparation d'alkyl-13α gonanes répondant à la formule générale I:

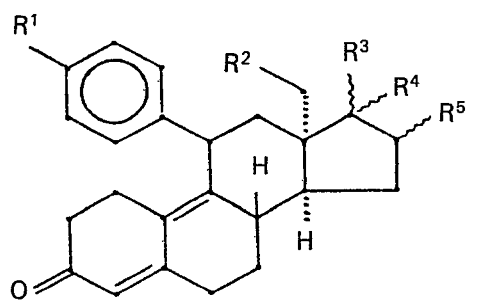

(I)

dans laquelle

$R^1$ représente un radical $-N{<}{R^{I} \atop R^{II}}$ dont les symboles $R^I$ et $R^{II}$ représentent chacun l'hydrogène ou un alkyle contenant de 1 à 4 atomes de carbone ou forment ensemble et avec l'atome N un cycle saturé à 5 ou 6 maillons, ce cycle pouvant contenir en outre, en plus de N, un hétéroatome supplémentaire, tel que O, N ou S, ou représente un radical N-oxyde tertiaire correspondant ou un sel d'addition d'acide correspondant, et $R^1$ peut également représenter un radical $-OR^{III}$ dans lequel $R^{III}$ désigne un radical méthyle, éthyle, propyle, méthoxyphényle, allyle ou diméthylamino-2 éthyle,

$R^2$ représente un atome d'hydrogène ou un radical méthyle ou éthyle,

$R^3$ représente:

– un radical $-(CH_2)_n-CH_3$ dans lequel n désigne un nombre de 0 à 4,

– un radical $-(CH_2)_n-CH_2-O(S)R^{IV}$ dans lequel n désigne un nombre de 0 à 4 et $R^{IV}$ représente l'hydrogène ou un alkyle contenant de 1 à 4 atomes de carbone,

– un radical $-CH=CH-(CH_2)_n-OR^V$ dans lequel n désigne un nombre de 1 à 4 et $R^V$ représente l'hydrogène, ou un alkyle ou un alcanoyle contenant chacun de 1 à 4 atomes de carbone,

– un radical $-C \equiv C-X$ dans lequel X représente l'hydrogène, un alkyle contenant de 1 à 4 atomes de carbone ou un halogène,

– un radical $-(CH_2)_n-CH_2CN$ dans lequel n désigne un nombre de 0 à 3 ou

– un radical $-\overset{\overset{O}{\|}}{C}-CH_2Y$ dans lequel Y représente l'hydrogène ou un radical $OR^V$ dont le symbole $R^V$ a la signification qui lui a été donnée ci-dessus,

$R^4$ représente un hydroxy, ou un alcoxy ou un alcanoyloxy contenant chacun de 1 à 4 atomes de carbone, ou encore $R^3$ et $R^4$ forment ensemble un radical:

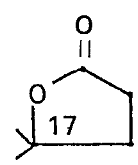

le substituant $R^3$ ayant la configuration α et le substituant $R^4$ la configuration β, ou inversement, sur le squelette stéroïdique, et

$R^5$ représente un atome d'hydrogène ou un radical alkyle qui contient de 1 à 4 atomes de carbone et qui a la configuration α ou la configuration β, procédé caractérisé en ce qu'on irradie par un rayonnement ultra-violet un composé répondant à la formule générale II:

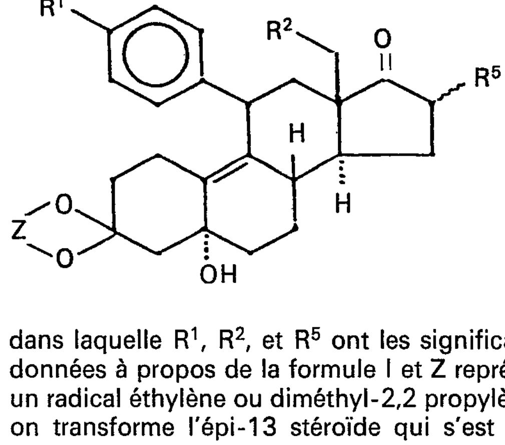

(II),

dans laquelle $R^1$, $R^2$, et $R^5$ ont les significations données à propos de la formule I et Z représente un radical éthylène ou diméthyl-2,2 propylène, et on transforme l'épi-13 stéroïde qui s'est formé

sous l'action du rayonnement ultra-violet et qui répond à la formule générale III:

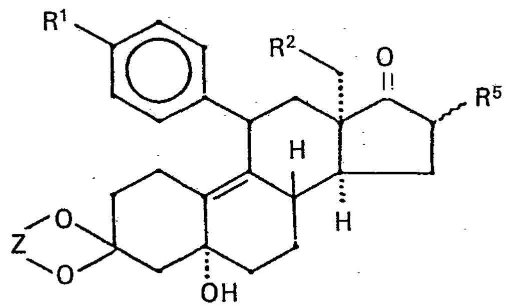

(III)

dans laquelle $R^1$, $R^2$, $R^5$ et Z ont les significations données à propos de la formule II, en appliquant des méthodes connues, par addition nucléophile sur le radical cétonique en 17, coupure du radical acétal protecteur en 3 et enlèvement d'eau en position 4, 5, en composés de formule générale I.

**Claims for the Contracting States BE, CH, LI, DE, FR, GB, IT, LU, NL, SE**

1. 13α-Alkyl gonanes of general formula I

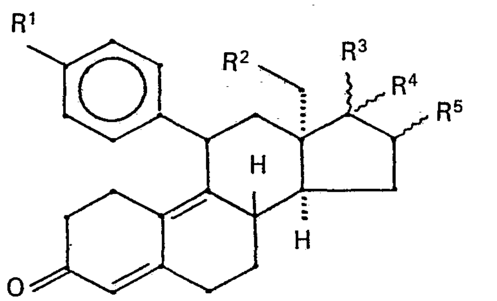

(I)

wherein

$R^1$ means $-N\genfrac{}{}{0pt}{}{R^{I}}{R^{II}}$ wherein $R^I$ and $R^{II}$ are hydrogen or alkyl of 1–4 carbon atoms, or $R^I$ and $R^{II}$ with inclusion of N mean a saturated 5- or 6-membered ring, wherein the ring can contain, besides N, still another hetero atom, such as O, N, S, as well as the corresponding tertiary N-oxides and the acid addition salts, $OR^{III}$ wherein $R^{III}$ means methyl, ethyl, propyl, methoxyphenyl, allyl, or β-dimethylaminoethyl,

$R^2$ means a hydrogen atom, a methyl group or an ethyl group,

$R^3$ means $-(CH_2)_n-CH_3$ with n=0–4,

$-(CH_2)_n-CH_2-O(S)R^{IV}$ with n=0–4, and $R^{IV}$ meaning hydrogen or alkyl of 1–4 carbon atoms,

$-CH=CH-(CH_2)_n-OR^V$ with n=1–4, and $R^V$ meaning hydrogen, alkyl, or alkanoyl of respectively 1–4 carbon atoms,

$-C{\equiv}C-X$ wherein X means hydrogen, alkyl of 1–4 carbon atoms, or halogen,

$-(CH_2)_n-CH_2CN$ with n=0–3 or

$-\overset{O}{\overset{\|}{C}}-CH_2Y$ wherein Y means hydrogen or $OR^V$,

$R^4$ is hydroxy, alkoxy, or alkanoyloxy of respectively 1–4 carbon atoms or

$R^3/R^4$ means 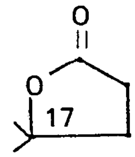

wherein $R^3$ is in the α-position and $R^4$ is in the β-position, or $R^3$ is in the β-position and $R^4$ is in the α-position with respect to the steroid skeleton, and

$R^5$ is a hydrogen atom or an α- or β-positioned alkyl group of 1–4 carbon atoms.

2. 11β-(4-Dimethylaminophenyl)- 17α-ethynyl-17β-hydroxy-13α- methyl-4,9-gonadien-3-one and 11β-(4-dimethylaminophenyl)- 17β-ethynyl-17α-hydroxy-13α- methyl-4,9-gonadien-3-one.

3. 11β-(4-Dimethylaminophenyl)-17β-hydroxy-13α- methyl-17α-propynyl- 4,9-gonadien-3-one and 11β-(4-dimethylaminophenyl)- 17α-hydroxy-13α-methyl-17β- propynyl-4,9-gonadien-3-one.

4. 11β-(Dimethylaminophenyl)-17α-hydroxy-13α-methyl-18,19-dinor-4,9-pregnadiene-3,20-dione and 17α-acetoxy-11β- (4-dimethylaminophenyl)-13α-methyl-18,19-dinor-4,9-pregnadiene-3,20-dione.

5. 11β-(4-Diethylaminophenyl)- 17α- (3-hydroxypropyl)- 17β-hydroxy-13α-methyl-4,9-gonadien-3-one and 11β-(4-diethylaminophenyl)- 17β- (3-hydroxypropyl)- 17α-hydroxy-13α-methyl- 4,9-gonadien-3-one.

6. 11β-(4-Dimethylaminophenyl)- 17β-hydroxy-17α- (3-hydroxypropyl)-13α-methyl- 4,9-gonadien-3-one and 11β-(4-dimethylaminophenyl)- 17α-hydroxy-17β- (3-hydroxypropyl)- 13α-methyl-4,9-gonadien-3-one.

7. 17β-Ethynyl-17α-hydroxy-11β- (4-methoxyphenyl)- 13α-methyl-4,9-gonadien-3-one.

8. 11β-(4-Dimethylaminophenyl)-17β-ethynyl-13α- ethyl-17α-hydroxy- 4,9-gonadien-3-one and 17α-acetoxy-11β- (4-dimethylaminophenyl)-13α-ethyl-18,19-dinor- 4,9-pregnadiene-3,20-dione.

9. 11β-(4-Dimethylaminophenyl)- 17α-hydroxy-17β- (3-hydroxy-1(Z)-propenyl)- 13α-methyl-4,9-gonadien-3-one.

10. 11β-(4-Dimethylaminophenyl)- 17β-ethynyl-16β- ethyl-17α-hydroxy-13α-methyl-4,9-gonadien- 3-one.

11. 17β-Cyanomethyl-11β- (4-dimethylaminophenyl)- 17α-hydroxy-13α- methyl-4,9-gonadien-3-one.

12. Pharmaceutical preparations characterized in that they contain compounds according to claims 1–11.

13. Process for the preparation of 13α-alkyl gonanes of general Formula I

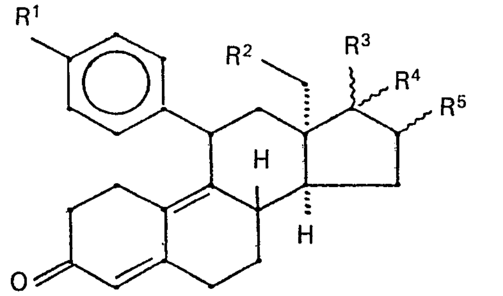

(I),

wherein $R^1$, $R^2$, $R^3$, $R^4$, and $R^5$ have the meanings given in claim 1, characterized in that a compound of general Formula II

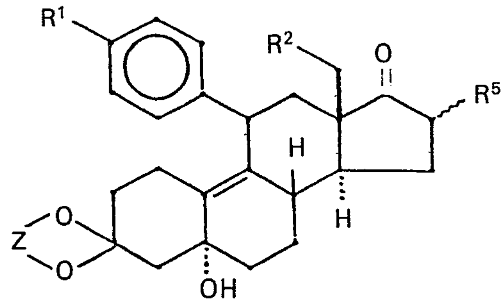

(II),

wherein

$R^1$, $R^2$, and $R^5$ have the meanings indicated in Formula I and

Z is an ethylene or 2,2-dimethylpropylene group, is irradiated with ultraviolet light, and the 13-episteroid, obtained by UV irradiation, of general Formula III

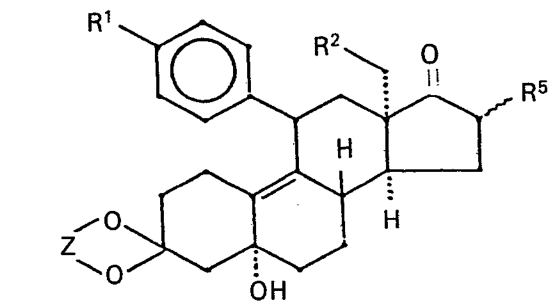

wherein $R^1$, $R^2$, $R^5$, and Z have the meanings given in Formula II, is converted into the compounds of general Formula I in accordance with methods known per se by nucleophilic addition to the 17-ketone, cleavage of the 3-ketal blockage, and splitting off water from the 4,5-position.

14. 13-Episteroids of general formula III

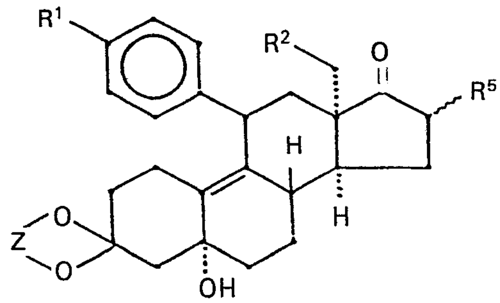

(III)

wherein

$R^1$ is $-N\genfrac{}{}{0pt}{}{R^{I}}{R^{II}}$ wherein $R^I$ and $R^{II}$ are hydrogen or alkyl of 1–4 carbon atoms, or $R^I$ and $R^{II}$ with inclusion of N mean a saturated 5- or 6-membered ring, wherein the ring can contain, besides N, still another hetero atom, such as O, N, S, as well as the corresponding tertiary N-oxides and the acid addition salts, $OR^{III}$ wherein $R^{III}$ means methyl, ethyl, propyl, methoxyphenyl, allyl, or β-dimethylaminoethyl,

$R^2$ means a hydrogen atom, a methyl group or an ethyl group,

$R^5$ means a hydrogen atom or an α- or β-positioned alkyl group of 1–4 carbon atoms, and

Z is an ethylene or 2,2-dimethylpropylene group.

**Claim for the Contracting State AT**

Process for the preparation of 13α-alkylgonanes of the general formula I

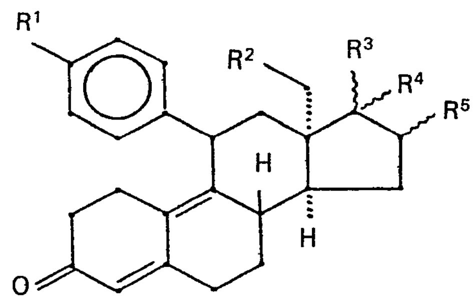

(I)

wherein

$R^1$ represents $-N\genfrac{}{}{0pt}{}{R^{I}}{R^{II}}$, wherein $R^I$ and $R^{II}$ represent hydrogen or alkyl having from 1 to 4 carbon atoms, or $R^I$ and $R^{II}$, with the inclusion of N, represent a saturated 5- or 6-membered ring, wherein the ring can contain, in addition to N, a further hetero atom, such as O, N or S, as well as the corresponding tertiary N-oxides and the acid addition salts, or represents $-OR^{III}$, wherein $R^{III}$ represents methyl, ethyl, propyl, methoxyphenyl, allyl or β-dimethylaminoethyl,

$R^2$ represents a hydrogen atom, a methyl group or an ethyl group, $R^3$ represents

$-(CH_2)_n-CH_3$, where n=0–4,

$-(CH_2)_n-CH_2-O(S)R^{IV}$, where n=0–4 and $R^{IV}$ represents hydrogen or alkyl having from 1 to 4 carbon atoms,

$-CH=CH-(CH_2)_n-OR^V$, where n=1–4 and $R^V$ represents hydrogen, alkyl or alkanoyl each having from 1 to 4 carbon atoms,

$-C\equiv C-X$, where X represents hydrogen, alkyl having from 1 to 4 carbon atoms, or halogen,

$-(CH_2)_n-CH_2CN$, where n=0–3, or

$-\overset{\displaystyle O}{\overset{\|}{C}}-CH_2Y$, where Y represents hydrogen or $OR^V$, where $R^V$ has the meanings given above,

$R^4$ represents hydroxy, alkoxy or alkanoyloxy each having from 1 to 4 carbon atoms, or

$R^3/R^4$ represents 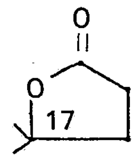

wherein $R^3$ is in the α-position and $R^4$ is in the β-position, or $R^3$ is in the β-position and $R^4$ is in the α-position with respect to the steroid skeleton, and

$R^5$ represents a hydrogen atom or an alkyl group in the α- or β-position having from 1 to 4 carbon atoms, characterised in that a compound of the general formula II

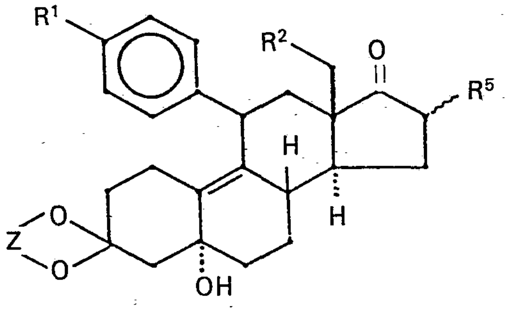

(II),

wherein $R^1$, $R^2$ and $R^5$ have the meanings given in formula I and Z is an ethylene or 2,2-dimethylpropylene group, is irradiated with ultraviolet light, and the 13-episteroid, obtained by UV irradiation, of the general formula III

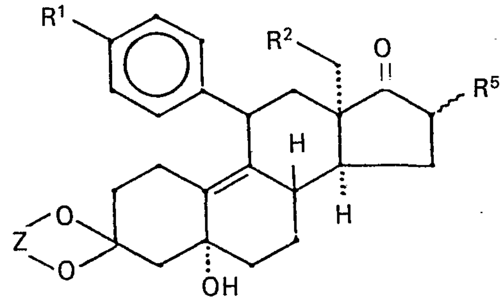

(III),

wherein $R^1$, $R^2$, $R^5$ and Z have the meanings given in formula II, is converted into the compounds of the general formula I in accordance with methods known per se by nucleophilic addition to the 17-ketone, cleavage of the 3-ketal protective group, and splitting off of water from the 4,5-position.